# EUROPEAN PATENT APPLICATION

(11) **EP 0 905 241 A1**
(43) Date of publication of application: **31.03.1999**
(21) Application number: 98104216.1
(22) Date of filing: 10.03.1998
(51) Int. Cl.: C12N 15/19, C07K 14/52, A61K 38/19, A61K 38/48, G01N 33/68

(54) **Amino-terminally truncated c-c chemokines as chemokine antagonists**

(30) Priority: 19.12.1997 EP 97122471; 29.09.1997 EP 97116863
(71) Applicant: APPLIED RESEARCH SYSTEMS ARS HOLDING N.V., Curaçao (AN)
(72) Inventor: Proost, Paul, 3001 Heverlee-Leuven (BE); Struyf, Sofie, 2841 Rumst (BE); Van Damme, Jo, 1000 Brussels (BE)
(74) Representative: Pieraccioli, Daniele

(57) **Abstract**

Amino-terminally truncated C-C chemokines, having chemokine antagonistic activity, are described. In particular, we refer to amino-terminally truncated C-C chemokines missing up to 5 amino-terminally amino acids and having a chemokine antagonistic activity. More particularly, MCP-2 (6-76) and RANTES (3-68) are described. The cDNA sequences encoding them, their use in therapy and/or in diagnosis of the diseases, in which an antagonistic activity of the chemokine effects is required, as well as pharmaceutical compositions comprising them.

## Description

### FIELD OF THE INVENTION

The present invention relates to amino-terminally truncated C-C chemokines, having chemokine antagonistic activity. In particular, we refer to amino-terminally truncated C-C chemokines missing up to 5 amino-terminally amino acids and having a chemokine antagonistic activity. More particularly, MCP-2 (6-76) and RANTES (3-68) are described, as well as cDNA sequences encoding them, their use in therapy and/or in diagnosis of the diseases, in which an antagonistic activity of the chemokine effects is required, and pharmaceutical compositions comprising them.

### BACKGROUND OF THE INVENTION

Chemokines constitute a family of small pro-inflammatory cytokines with leukocyte chemotactic and activating properties. Depending on the position of the first cysteines, the chemokine family can be divided in C-C, C-X-C and C-X₃-C chemokines (Baggiolini et al., *Ann. Rev. Immunol*., 55, 97-179, 1994; Baggiolini et al., *Ann. Rev. Immunol*., 15, 675-705, 1997; and Taub et al., *Cytokine & Growth Factor Reviews*, 7, 335-76, 1996).

Many C-X-C chemokines such as interleukin-8 (IL-8) are chemotactic for neutrophils, while C-C chemokines, such as monocyte chemotactic protein-3 (MCP-3), are active on a variety of leukocytes including monocytes, lymphocytes, eosinophils, basophils, NK cells and dendritic cells.

The NH₂ terminal domain of chemokines is involved in receptor-binding and NH₂-terminal processing can either activate chemokines or render chemokines completely inactive.

The C-X-C chemokine platelet basic protein becomes a neutrophil chemotactic peptide (NAP-2) only after removal of the 24 NH₂-terminal residues (Walz et al., *Biochem. Biophys. Res. Commun*., 159, 969-75, 1989; and Van Damme et al., *Eur. J. Immunol*., 20, 2113-8, 1990).

Deletion of up to 8 NH₂-terminal residues from IL-8 results in an enhanced chemotactic activity, but further cleavage of the Glu-Leu-Arg motif, which is located in front of the first Cys in all neutrophil chemotactic C-X-C chemokines, causes complete inactivation (Clark-Lewis I. et al., *J. Biol. Chem*., 266, 23128-23134, 1991).

Similar NH₂-terminal proteolysis (up to 8 amino acids) of another C-X-C chemokine, granulocyte chemotactic protein-2 (GCP-2), has no effect on the neutrophil chemotactic activity (Proost P.et al., *Biochemistry*, 32, 10170-10177, 1993).

The synthetical C-C chemokines MCP-1, MCP-3 and RANTES missing the 8 to 9 NH₂-terminal amino acids are inactive on monocytes and are useful as receptor antagonists (Gong J. et al., *J. Biol. Chem*., 271, 10521-7, 1996; and Gong G. et al., *J. Exp. Med*., 631-40, 1995).

Extension of RANTES with one methionine results in complete inactivation of the molecule and Met-RANTES behaves as an antagonist for the authentic RANTES (Proudfoot A.E. et al., *J. Biol. Chem*., 271, 2599-2603, 1996).

### DESCRIPTION OF THE INVENTION

The main object of the present invention are amino-terminally truncated C-C chemokines, having chemokine antagonistic activity. In particular, we refer to amino-terminally truncated C-C chemokines missing up to 5 amino-terminally amino acids and having a chemokine antagonistic activity. A non-limiting list of C-C chemokines include: MCP-1, MCP-2, MCP-3, MCP-4, MCP-5, RANTES, MDC, MIP-1α and MIP-1β.

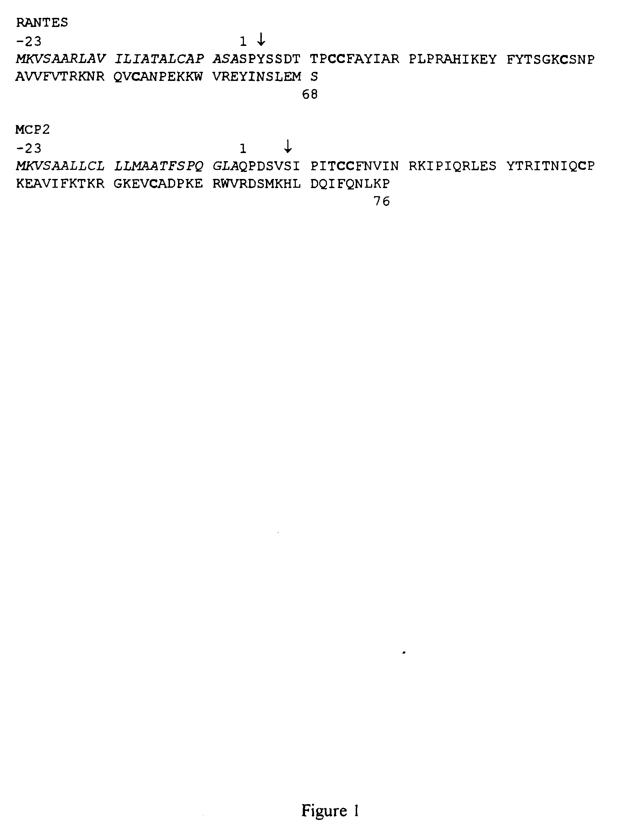

More particularly, one object of the present invention is MCP-2 (6-76), which is MCP-2 missing the 5 NH₂-terminal amino acids, as shown in Figure 1.

Another object of the present invention is RANTES (3-68), which is RANTES missing the first 2 amino acids, as shown in Figure 1.

Such amino-terminally truncated C-C chemokines of the invention can be in a glycosylated or non-glycosylated form.

Another object of the invention are the DNA molecules comprising the DNA sequences coding for the amino-terminally truncated chemokines of the invention, including nucleotide sequences substantially the same.

"Nucleotide sequences substantially the same" includes all other nucleic acid sequences which, by virtue of the degeneracy of the genetic code, also code for the given amino acid sequences.

The invention also includes expression vectors which comprise the above DNAs, host-cells transformed with such vectors and a process of preparation of such amino-terminally truncated chemokines of the invention, through the culture in appropriate culture media of said transformed cells.

The DNA sequence coding for the proteins of the invention can be inserted and ligated into a suitable plasmid. Once formed, the expression vector is introduced into a suitable host cell, which then expresses the vector(s) to yield the desired protein.

Expression of any of the recombinant proteins of the invention as mentioned herein can be effected in eukaryotic cells (e.g. yeasts, insect or mammalian cells) or prokaryotic cells, using the appropriate expression vectors. Any method known in the art can be employed.

For example the DNA molecules coding for the proteins obtained by any of the above methods are inserted into appropriately constructed expression vectors by techniques well known in the art (see Sambrook et al, 1989). Double stranded cDNA is linked to plasmid vectors by homopolymeric tailing or by restriction linking involving the use of synthetic DNA linkers or blunt-ended ligation techniques: DNA ligases are used to ligate the DNA molecules and undesirable joining is avoided by treatment with alkaline phosphatase.

In order to be capable of expressing the desired protein, an expression vector should also comprise specific nucleotide sequences containing transcriptional and translational regulatory information linked to the DNA coding the desired protein in such a way as to permit gene expression and production of the protein. First in order for the gene to be transcribed, it must be preceded by a promoter recognizable by RNA polymerase, to which the polymerase binds and thus initiates the transcription process. There are a variety of such promoters in use, which work with different efficiencies (strong and weak promoters).

For eukaryotic hosts, different transcriptional and translational regulatory sequences may be employed, depending on the nature of the host. They may be derived form viral sources, such as adenovirus, bovine papilloma virus, Simian virus or the like, where the regulatory signals are associated with a particular gene which has a high level of expression. Examples are the TK promoter of the Herpes virus, the SV40 early promoter, the yeast gal4 gene promoter, etc. Transcriptional initiation regulatory signals may be selected which allow for repression and activation, so that expression of the genes can be modulated.

The DNA molecule comprising the nucleotide sequence coding for the protein of the invention is inserted into vector(s), having the operably linked transcriptional and translational regulatory signals, which is capable of integrating the desired gene sequences into the host cell.

The cells which have been stably transformed by the introduced DNA can be selected by also introducing one or more markers which allow for selection of host cells which contain the expression vector. The marker may also provide for phototrophy to a auxotropic host, biocide resistance, e.g. antibiotics, or heavy metals such as copper, or the like. The selectable marker gene can either be directly linked to the DNA gene sequences to be expressed, or introduced into the same cell by co-transfection. Additional elements may also be needed for optimal synthesis of proteins of the invention.

Factors of importance in selecting a particular plasmid or viral vector include: the ease with which recipient cells, that contain the vector may be recognized and selected from those recipient cells which do not contain the vector; the number of copies of the vector which are desired in a particular host; and whether it is desirable to be able to "shuttle" the vector between host cells of different species.

Once the vector(s) or DNA sequence containing the construct(s) has been prepared for expression the DNA construct(s) may be introduced into an appropriate host cell by any of a variety of suitable means: transformation, transfection, conjugation, protoplast fusion, electroporation, calcium phosphate-precipitation, direct microinjection, etc.

Host cells may be either prokaryotic or eukaryotic. Preferred are eukaryotic hosts, e.g. mammalian cells, such as human, monkey, mouse, and Chinese hamster ovary (CHO) cells, because they provide post-translational modifications to protein molecules, including correct folding or glycosylation at correct sites. Also yeast cells can carry out post-translational peptide modifications including glycosylation. A number of recombinant DNA strategies exist which utilize strong promoter sequences and high copy number of plasmids which can be utilized for production of the desired proteins in yeast. Yeast recognizes leader sequences on cloned mammalian gene products and secretes peptides bearing leader sequences (i.e., pre-peptides).

After the introduction of the vector(s), the host cells are grown in a selective medium, which selects for the growth of vector-containing cells. Expression of the cloned gene sequence(s) results in the production of the desired proteins.

The amino-terminally truncated chemokines of the invention may be prepared by any other well known procedure in the art, in particular, by the well established chemical synthesis procedures, utilizing automated solid-phase peptide synthesizers followed by chromatographic purification.

The chemokines of the invention may, for example, be synthesized by Fmoc (9-fluorenylmethoxycarbonyl), tBoc (t-butoxycarbonyl) or any other comparable chemical synthesis with or without appropriate side-chain protection groups on the different amino acids. The amino acids with or without appropriate side-chain protection groups are preactivated - e.g. with HBTU/HOBt [2-(1H-Benzotriazole-1yl)-1,1,3,3-tetramethyluromium hexafluorophosphate/1-hydroxybenzotriazole) - and coupled to the growing peptide chain. Before the addition of the following residue, the protection group (e.g. Fmoc) is removed from the α-amino group. After synthesis, all protection groups are removed, the intact full length peptides are purified and chemically or enzymatically folded (including the formation of disulphide bridges between cysteines) into the corresponding chemokines of the invention.

Purification of the natural, synthetic or recombinant proteins is carried out by any one of the methods known for this purpose, i.e. any conventional procedure involving extraction, precipitation, chromatography, electrophoresis, or the like (see for example Proost P. et al., *Methods: A companion to Methods in Enzymol*., 10, 82, 1996). A further purification procedure that may be used in preference for purifying the protein of the invention is affinity chromatography using monoclonal antibodies, affinity or heparin, which bind the target protein and which are produced and immobilized on a gel matrix contained within a column. Impure preparations containing the proteins are passed through the column. The protein will be bound to the column by the specific antibody while the impurities will pass through. After washing, the protein is eluted from the gel by a change in pH or ionic strength.

The amino-terminally truncated chemokines of the invention are useful in the therapy and/or diagnosis of the diseases, in which an antagonistic activity of the chemokine effects is required. Examples of such diseases include: inflammatory diseases, angiogenesis- and hematopoiesis-related diseases, tumors, infectious diseases, including HIV, auto-immune diseases, atherosclerosis, pulmonary diseases and skin disorders.

Therefore, in a further aspect, the present invention provides the use of the protein of the invention in the manufacture of a medicament for the treatment of the above-mentioned diseases.

The medicament is preferably presented in the form of a pharmaceutical composition comprising the proteins of the invention together with one or more pharmaceutically acceptable carriers and/or excipients. Such pharmaceutical compositions form yet a further aspect of the present invention.

It has also been found that CD26/DPP IV is able to generate NH₂-terminally truncated chemokines in vitro. RANTES is the first cytokine reported whose biological activity can be modified by CD26/DPP IV.

Therefore, another object of the present invention is the use of CD26/DPP IV in the therapy and/or diagnosis of the diseases, in which an antagonistic activity of the chemokine effects is required, with particular focus on inflammatory, immune and infectious diseases.

Since this represents the first example of an identified mechanism for endogeneously regulated chemokine modification into an antagonist, similar physiological processing, but mediated by other factors (proteases), must be responsible for the MCP-2 truncation into MCP-2(6-76). The use of such factors (proteases) in the therapy and/or diagnosis of the above diseases is also included in this invention.

The invention will now be described by means of the following Examples, which should not be construed as in any way limiting the present invention. The Examples will refer to the Figures specified here below.

### DESCRIPTION OF THE FIGURES

Figure 1: it shows the amino acid sequences of MCP-2 and RANTES. Signal sequences are reported in *italics*, whereas C -residues are in **bold**. Arrows indicate the first amino acids of the amino-terminally truncated chemokines of the invention.

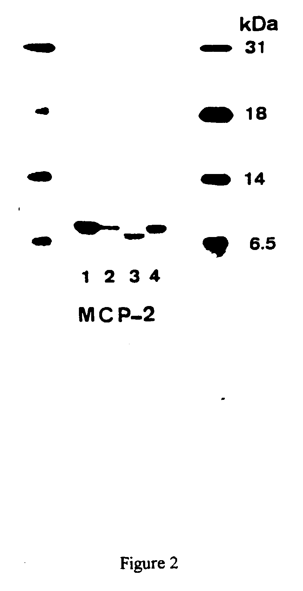

Figure 2: SDS-PAGE of amino-terminally truncated MCP-2(6-76):
   lane 1: natural MCP-2 (1-76, 100 ng/ml);
   lane 2: natural MCP-2 (1-76, 30 ng/ml);
   lane 3: natural MCP-2 (6-76, 30 ng/ml); and
   lane 4: synthetic MCP-2 (1-76, 60 ng/ml).
      Gels were run under reducing conditions and proteins were stained with silver.

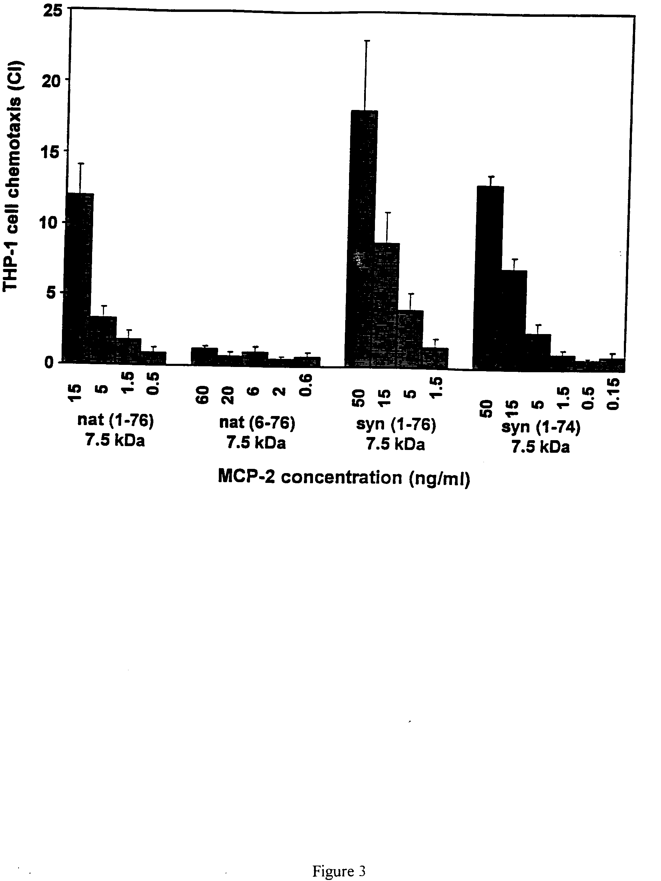

Figure 3: it shows a comparison of the chemotactic potency of modified MCP-2 forms. Intact natural and synthetic MCP-2(1-76), NH₂-terminally truncated natural MCP-2(6-76) and COOH-terminally truncated synthetic MCP-2(1-74) were tested for chemotactic activity on THP-1 cells. Results represent the mean CI ± SEM from four or more independent experiments.

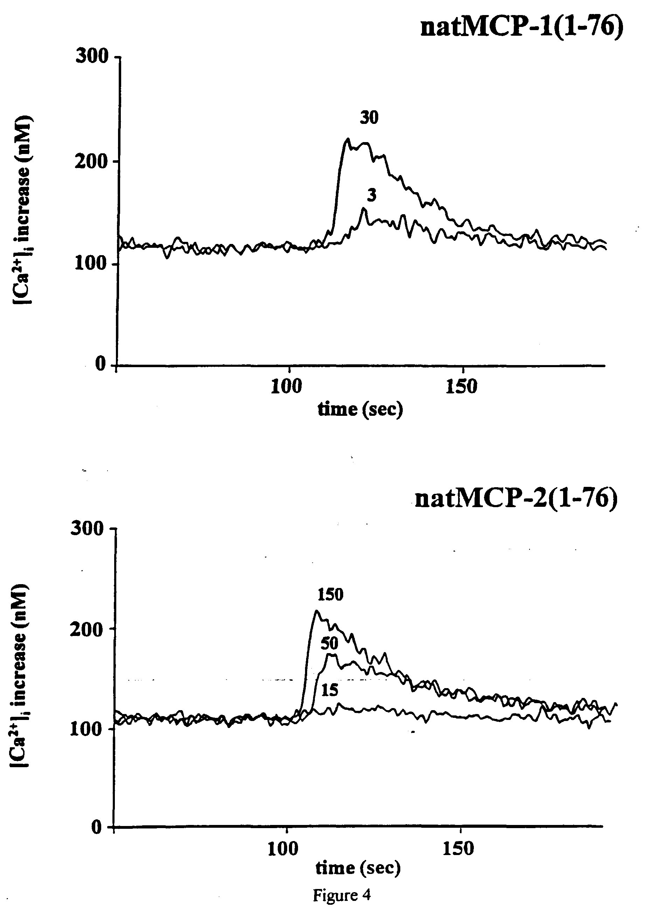

Figure 4: Natural MCP-2 is a weaker agonist than MCP-1 to mobilize calcium in monocytes. Intact MCP-2 (15, 50 and 150 ng/ml) dose-dependently increases the [Ca²⁺]ᵢ in THP-1 cells. The result of one representative experiment out of two is shown.

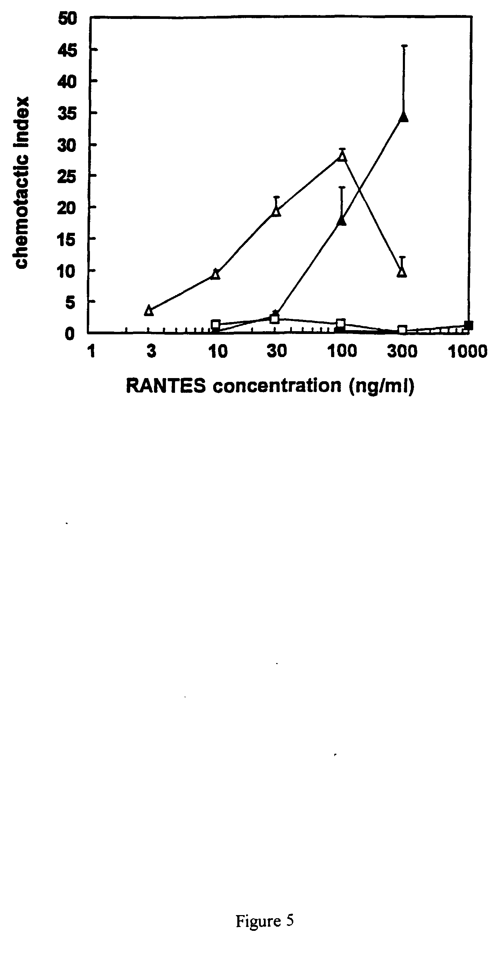

Figure 5: The chemotactic potencies of intact and NH₂-terminally truncated forms of natural or recombinant RANTES for monocytic THP-1 cells were compared in the Boyden microchamber assay: Natural RANTES(1-68) (Δ), natural, truncated RANTES(3-68) (□), intact recombinant RANTES(1-68) (Δ) and CD26/DPP IV cleaved recombinant RANTES(3-68) (■). Results represent the mean chemotactic index ± SEM of four or more independent experiments.

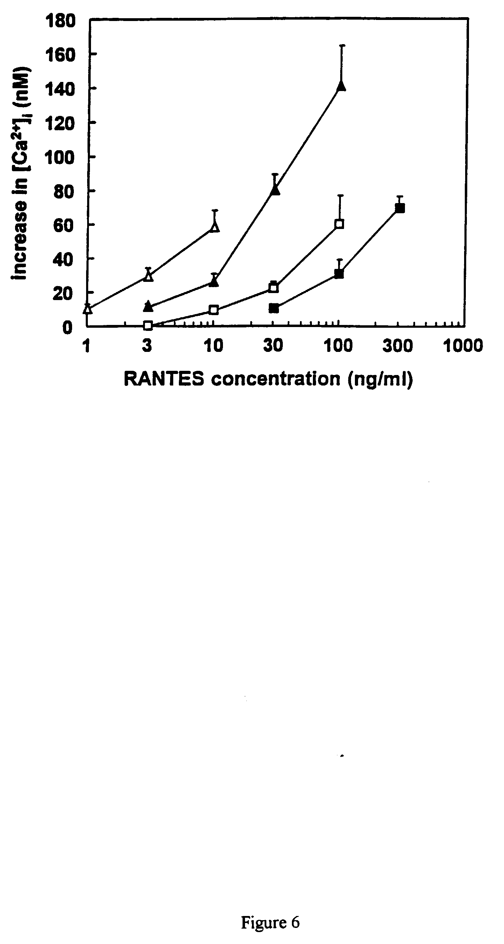

Figure 6: Effect of natural RANTES(3-68) (□), natural RANTES(1-68) (Δ), recombinant RANTES(1-68) (▲) and recombinant CD26/DPP IV treated RANTES(3-68) (■) on the [Ca²⁺]ᵢ in THP-1 cells. Results represent the mean increase in [Ca²⁺]ᵢ ± SEM of three or more independent experiments.

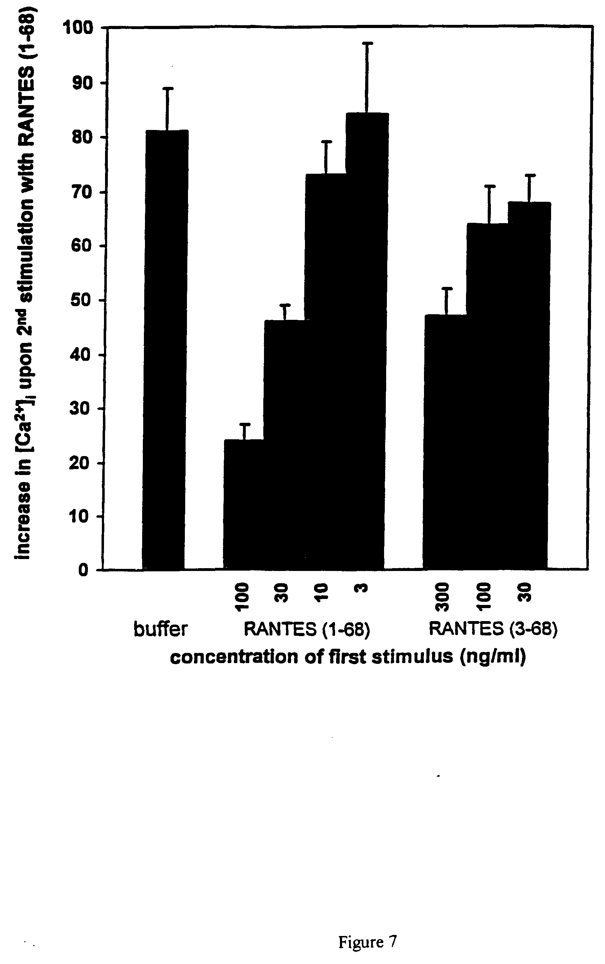

Figure 7: It shows the desensitization of the Ca²⁺-mobilizing activity of intact RANTES(1-68) by RANTES(3-68). THP-1 cells were first stimulated with buffer or different concentrations of recombinant RANTES(1-68) or RANTES(3-68). Results represent the mean ± SEM (three or more independent experiments) increase in [Ca²⁺]ᵢ in response to 30 ng/ml of intact recombinant RANTES as a second stimulus.

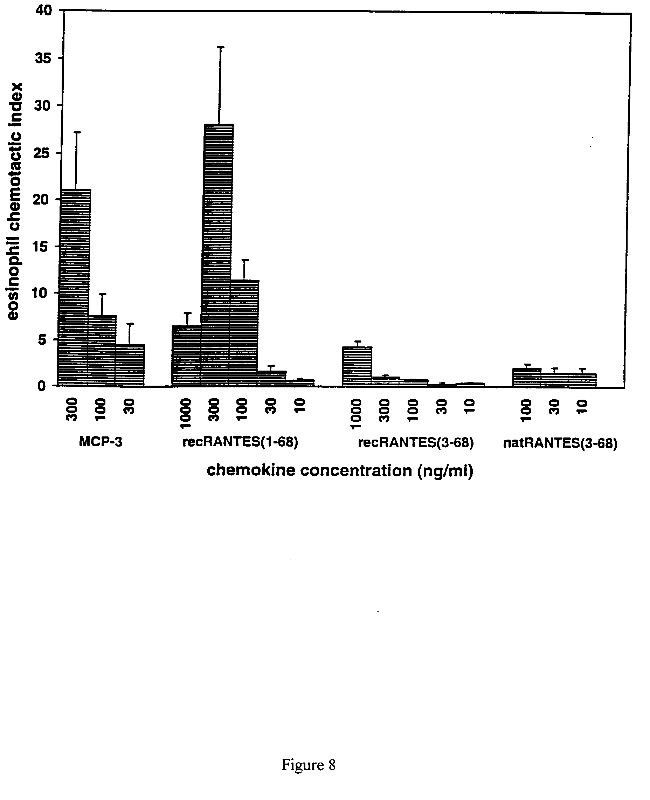

Figure 8: Comparison of the chemotactic potency of truncated RANTES(3-68) with intact RANTES(1-68). The eosinophilic granulocyte chemotactic activity of natural and recombinant truncated RANTES, intact RANTES and synthetic MCP-3 was determined in the microchamber assay. Results represent the mean chemotactic index (CI) ± SEM of two or more independent experiments (each performed in triplicate).

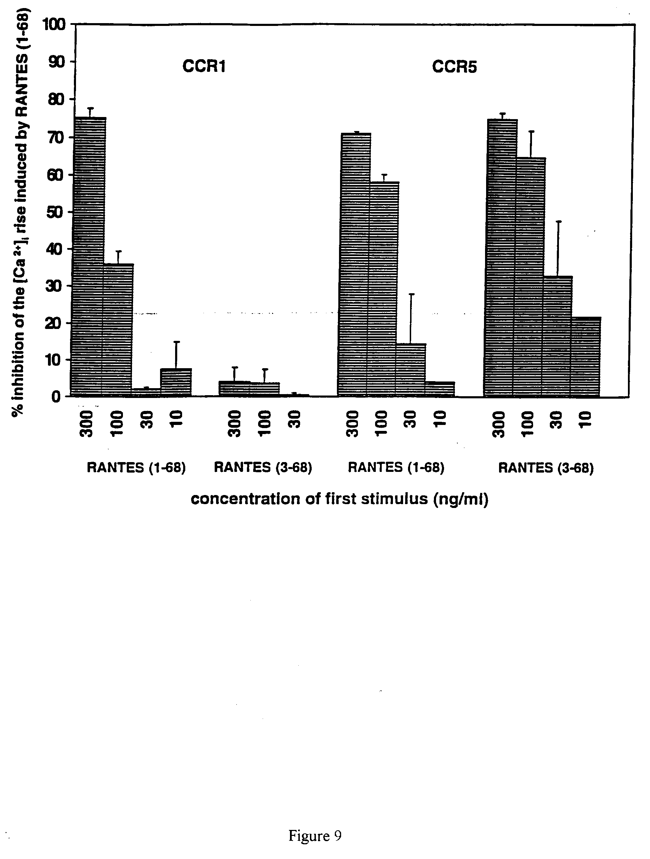

Figure 9: Desensitization of calcium mobilization by intact RANTES in CCR transfectants. Calcium mobilization experiments were performed in HOS cells transfected with CD4 and the CC chemokine receptors CCR1 or CCR5. Cells were first stimulated with different concentrations of intact or truncated RANTES, followed by stimulation with 100 ng/ml of intact RANTES. The percentage inhibition of the [Ca²⁺]ᵢ increase induced by the second stimulus is shown. This percentage was calculated by comparing the response of 100 ng/ml of intact RANTES after addition of RANTES(1-68) or RANTES(3-68) with the reponse after stimulation with buffer (100%). Results represent the mean percentage inhibition ± SEM of two or more experiments.

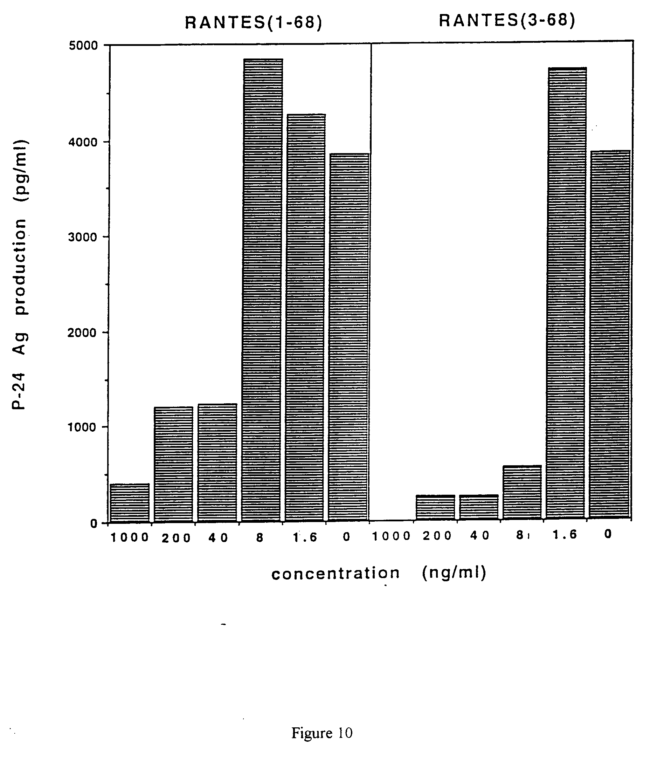

Figure 10: Potent inhibitory effect of RANTES (3-68) on infection of mononuclear cells by HIV-1. PHA-activated PBMC were infected with M-tropic HIV-1 Ba-L strain in the presence of various concentrations of RANTES (1-68) or RANTES (3-68) (0 to 1,000 ng/ml added at the time of infection). After ten days virus yields were monitored in the cell supernatant by a p-24 Ag ELISA (one representative experiment out of four is shown).

### EXAMPLES

### EXAMPLE 1: Amino-terminally truncated MCP-2

### Materials and methods

### Chemokine and immunoassay

MCP-2 was synthesized and purified as described earlier (Proost P. et al., *Cytokine* 7(97), 1995).

Specific anti-human MCP-2 Ab were obtained from mice and affinity purified on a Sepharose column to which synthetic MCP-2 was coupled using the conditions provided by the manufacturer (CNBr activated Sepharose 4B, Pharmacia, Uppsala, Sweden).

ELISA plates were coated with the affinity purified anti-human MCP-2 and biotinylated anti-MCP-2 was used as the capturing Ab. The detection was performed with peroxidase labeled streptavidine and TMB. The detection limit for MCP ELISA was about 0.1 ng/ml.

### Production and purification of MCP-2

Monocyte chemotactic proteins were purified from peripheral blood mononuclear cell-derived conditioned medium from 132 blood donations obtained from Blood Transfusion Centers of Antwerp and Leuven (Proost P. et al., *Methods: A companion to Methods in Enzymol*., 10, 82, 1996).

Erythrocytes and granulocytes were removed by sedimentation in hydroxyethyl starch (Fresenius AG, Bad Homburg, Germany) and by gradient centrifugation in a sodium metrizoate solution (Lymphoprep; Nyegaard, Oslo Norway).

Mononuclear cells (60x10⁹ cells) were incubated (5x10⁶ cells/ml) with 10 µg/ml Con A and 2 µg/ml of LPS. After 48 to 120 h, conditioned medium was collected and kept at -20 °C until purification.

Natural MCP was purified in a four step purification procedure as previously described (Proost P. et al., *Methods: A companion to Methods in Enzymol*., 10, 82, 1996).

Briefly, the conditioned medium was concentrated on controlled pore glass or silicic acid and partially purified by affinity chromatography on a heparin-Sepharose column (Pharmacia).

Fractions containing MCP immunoreactivity were further purified by Mono S (Pharmacia) cation exchange chromatography and eluted in a NaCl gradient at pH 4.0.

Natural MCPs were purified to homogeneity through RP-HPLC on a C-8 Aquapore RP-300 column (Perkin Elmer, Norwalk CT) equilibrated with 0.1 % trifluoroacetic acid TFA). Proteins were eluted in an acetonitrile gradient.

### Biochemical characterization of MCP-forms by SDS-PAGE, amino acid sequence analysis and mass spectrometry

The purity of column fractions was examined by SDS-PAGE under reducing conditions on Tris/tricine gels (Proost P. et al., *Methods: A companion to Methods in Enzymol*., 10, 82, 1996). Proteins were stained with silver and the following relative molecular (*Mr*) markers were used: OVA (*Mr* 45,000), carbonic anhydrase (*Mr* 31,000), soybean trypsin inhibitor (*Mr* 21,500), β-lactoglobulin (*Mr* 18,400), lysozyme (*Mr* 14,400) and aprotinin (*Mr* 6,500).

The NH₂-terminal sequence of purified chemokines was determined by Edman degradation on a pulsed liquid 477A/120A protein sequencer (Perkin Elmer) with N-methylpiperidine as a coupling base. Blocked proteins were cleaved between Asp and Pro in 75 % formic acid for 50 h. The formic acid digest was sequenced without further purification.

The *Mr* of MCP-2 was determined by matrix-assisted laser desorption ionization/time of flight-mass spectrometry (MALDI/TOF-MS) (Micromass TofSpec, Manchester, UK). Alpha-cyano-4-hydroxycinnamic acid and cytochrome C were used as matrix and internal standard, respectively.

### Detection of chemotactic activity

MCP-2 was tested for its chemotactic potency on freshly purified monocytes (2x10⁶ cells/ml) or monocytic THP-1 cells (0.5x10⁶ cells/ml; 2 days after subcultivation) in the Boyden microchamber using polyvinylpyrrolidone-treated polycarbonate membranes with 5 µm pore size.

Samples and cells were diluted in HBSS (Life technologies/Gibco BRL, Paisley, Scotland) supplemented with 1 mg/ml human serum albumin (Red Cross Belgium). After 2 h incubation at 37 °C, the cells were fixed and stained with Diff-Quick staining solutions (Harleco, Gibbstown. NJ) and the cells that migrated through the membranes were counted microscopically in ten oil immersion fields at 500-fold magnification.

The chemotactic index (CI) of a sample (triplicates in each chamber) was calculated as the number of cells that migrated to the sample over the number of cells that migrated to control medium (Van Damme J. *J. Exp.Med*., 176, 59, 1992).

For desensitization experiments, cells were incubated with biologically inactive chemokine-variants for 10 min. at 37°C, before they were added to the upper well of the Boyden microchamber. The % inhibition of the CI was calculated using the CI from HBSS treated cells towards the sample as a reference value.

### Detection of intracellular Ca²⁺ concentrations

Intracellular calcium concentrations ([Ca²⁺ ]ᵢ) were measured as previously described (Wuyts et al., *Biochemistry*, 32, 2716, 1997). Purified monocytes or THP-1 cells (107 cells/ml) were incubated in Eagle's Minimum Essential Medium (EMEM, Gibco) + 0.5 % FCS with the fluorescent indicator fura-2 (fura-2/AM 2.5 µM; Molecular Probes Europe BV, Leiden, The Netherlands) and 0.01 % Pluronic F-127 (Sigma, St Louis MO).

After 30 min at 37 °C the cells were washed twice and resuspended at 106 cells/ml in HBSS with 1 mM Ca²⁺ and 0.1 % FCS (buffered with 10 mM Hepes/NaOH at pH 7.4). The cells were equilibrated at 37 °C for 10 min before fura-2 fluorescence was measured in a LS50B luminescence spectrophotometer (Perkin Elmer).

Upon excitation at 340 and 380 nm, fluorescence was detected at 510 nm. The [Ca²⁺ ]ᵢ was calculated from the Grynkiewicz equation (Grynkiewicz et al., *J. Biol. Chem*., 260, 3440, 1985). In order to determine *R*_{*max*} the cells were lysed with 50 µM digitonin. Subsequently, the pH was adjusted to 8.5 with 20 mM Tris and *R*_{*min*} was obtained by addition of 10 mM EGTA to the lysed cells. The *K*_{*d*} used was 224 nM.

For desensitization experiments, monocytes or THP-1 cells were first stimulated with buffer, chemokine or chemokine antagonist at different concentrations. As second stimulus, MCP-2 was used at a concentration inducing a significant increase in the [Ca²⁺ ]ᵢ after prestimulation with buffer. The second stimulus was applied 2 min after addition of the first stimulus. The percentage inhibition of the [Ca²⁺ ]ᵢ increase in response to the second stimulus was calculated comparing the signal alter prestimulation with chemokine or chemokine antagonist with the signal after addition of buffer.

### Results

### Isolation of post-translationally modified MCP-2 forms

A specific and sensitive ELISA was used to trace different MCP-2 forms produced by peripheral blood mononuclear cells stimulated with mitogen and endotoxin.

The conditioned medium was purified according to a standard isolation procedure (Proost P. et al., *Methods: A companion to Methods in Enzymol*., 10, 82, 1996), including adsorption to controlled pore glass and heparin Sepharose chromatography.

Subsequently, purification by FPLC mono S cation exchange chromatography was carried out and then a further purification step with C-8 RP HPLC was applied. Molecular masses were measured by SDS-PAGE and by MALDI/TOF-MS.

Different forms of MCP-2 were isolated: in addition to the authentic 7.5 kDa MCP-2(1-76), an NH₂-terminally truncated 7 kDa form of MCP-2 missing five residues [MCP-2(6-76)] was purified to homogeneity by RP-HPLC and identified by amino acid sequence analysis (Fig. 2). MALDI/TOF-MS (Table I) yielded a molecular mass of 8881 Da for intact MCP-2 (theoretical *Mr* of 8893 Da), whereas for the MCP-2(6-76) a molecular mass of 8365 Da was measured, confirming the deletion of the five NH₂-terminal amino acids (theoretical *Mr* of 8384 Da). Functional comparison of these natural MCP-2 forms in the THP-1 chemotaxis assay showed that intact MCP 2 is still active at 5 ng/ml, whereas truncated MCP-2(6-76) remains devoid of chemotactic activity when tested at a concentration range from 0.6 to 60 ng/ml (Fig. 3). Intact natural MCP-2 was also compared in potency with the synthetical MCP-2(1-76) and a COOH-terminally truncated synthetical form (Proost P. et al., *Cytokine* 7(97), 1995) missing two residues [MCP-2(1-74)].

The minimal effective chemotactic concentration of these forms was also found to be 5 ng/ml (Fig. 3). Although in chemotaxis assays the specific activity of natural intact MCP-1 and MCP-2 is comparable (Van Damme J, et al., *J. Exp. Med*., 176, 59, 1992), the calcium mobilizing capacity of MCP-2 is still a matter of debate.

However, in Ca²⁺ -mobilization experiments, the minimal effective dose for both natural or synthetic MCP-2(1-76) was 10-fold higher compared to that of natural intact MCP-1(1-76) (Fig. 4), whereas MCP-2(6-76) remained inactive.

Nevertheless, intact MCP-2 (50 ng/ml) was capable to desensitize for MCP-2 (15 ng/ml) and MCP-3 (10 ng/ml) yielding 52% and 45% inhibition, respectively.

Due to this lower specific activity of MCP-2 in Ca²⁺ assays, desensitization of chemotaxis by MCP-2(6-76) was performed in the Boyden microchamber. Since intact MCP-2 is reported to cross-desensitize with active MCP-1, MCP-2 and MCP 3 in the monocyte chemotaxis assay (Sozzani et al., *J. Immunol*., 152, 3615, 1994), we investigated whether natural, inactive MCP-2(6-76) could also desensitize for MCP-1, MCP-2, MCP-3 and RANTES (Table III). Pre-incubation of THP-I cells with 100 ng/ml of inactive MCP-2(6-76) could already significantly inhibit chemotaxis induced by 10 ng/ml of MCP-1 (63 %), 5 ng/ml of MCP-2 (75%), 30 ng/ml of MCP-3 (62 %) and 100 ng/ml of RANTES (75%). Moreover, chemotaxis in response to 3 times lower concentrations of the respective MCPs was completely (91-100 %) inhibited by 100 ng/ml MCP-2(6-76). Furthermore, at a concentration as low as 10 ng/ml, MCP-2(6-76) was still able to significantly inhibit the chemotactic activity induced by MCP-1 (3 ng/ml), MCP-2 (1.5 ng/ml) or MCP 3 (10 ng/ml) and RANTES (30 ng/ml). Taken together, MCP-2(6-76) is produced naturally, is inactive as a chemoattractant and antagonizes several C-C chemokines, the effect being most predominant for MCP-3.

**TABLE I**

| *Biochemical characterization of natural forms of MCP-2. NH2-terminal amino acid sequence analysis and comparison of the experimental (SDS-PAGE and MALDI/TOF-MS) and theoretical Mr of C-8 RP-HPLC purified natural MCP-isoforms.* | | | | |
|---|---|---|---|---|
| MCP-form | NH₂-terminal sequence | *Mr* (Da) | | |
| | | theoretical unglycosylated | SDS-PAGE | MALDI/TOF-MS |
| MCP-2 (1-76) | blocked | 8893 | 7500 | 8881 |
| MCP-2 (2-76) | SIPITCC | 8384 | 7000 | 8365 |

**TABLE II**

| *MCP-2(6-76) desensitizes the monocyte chemotactic responses of MCP-1, MCP-2 MCP-3 and RANTES in the microchamber.* | | | | |
|---|---|---|---|---|
| Chemokine ^{*a*} | Concentration | Antagonization of chemotactic response ^{*b*}^{*,*}^{*c*} | | % Inhibition of chemotaxis |
| | | buffer | 100 ng/ml MCP-2(6-76) | |
| MCP-1 | 10 | 22.3 ± 7.9 | 8.3 ± 3.8 | 63 ± 21 |
| | 3 | 15.0 ± 8.0 | 1.3 ± 0.3 | 99 ± 1.0 |
| MCP-2 | 5 | 36.0 ± 12.6 | 10.8 ± 6.1 | 75 ± 8.0 |
| | 1.5 | 6.7 ± 1.4 | 1.5 ± 0.3 | 91 ± 7.0 |
| MCP-3 | 30 | 13.2 ± 0.4 | 6.0 ± 4.0 | 62 ± 31 |
| | 10 | 3.0 ± 1.5 | <1 | 100 ± 0.0 |
| RANTES | 100 | 6.3 ± 0.8 | 2.6 ± 1.3 | 75 ± 19 |
| | 30 | 4.0 ± 0.8 | 1.5 ± 0.3 | 77 ± 16 |

| | | buffer | 10 ng/ml MCP-2 (6-76) | |
|---|---|---|---|---|
| MCP-1 | 10 | 12.7 ± 2.3 | 10.5 ± 3.8 | 24 ± 1.8 |
| | 3 | 7.5 ± 0.0 | 3.0 ± 0.3 | 69 ± 4.0 |
| MCP-2 | 5 | 38.0 ± 5.3 | 27.2 ± 4.9 | 30 ± 6.0 |
| | 1.5 | 18.3 ± 4.6 | 9.2 ± 1.4 | 45 ± 23 |
| MCP-3 | 30 | 13.2 ± 1.9 | 8.0 ± 1.0 | 37 ± 19 |
| | 10 | 7.7 ± 1.4 | 1.7 ± 0.3 | 90 ± 6.0 |
| RANTES | 100 | 5.5 ± 0.6 | 5.8 ± 0.9 | 17 ± 7.0 |
| | 30 | 3.2 ± 0.7 | 2.5 ± 0.5 | 39 ± 18 |

| | | | | |
|---|---|---|---|---|
| ^{a} MCP-1, MCP-2, MCP-3 or RANTES were added as chemoattractants to the lower wells. | | | | |
| ^{b} the upper wells of the microchamber were filled with THP-1 cells preincubated with MCP-2(6-76) or with buffer | | | | |
| ^{c} mean CI ± SEM of 3 independent experiments | | | | |

### EXAMPLE 2: Amino-terminally truncated RANTES

### Material and Methods

### Reagents

Natural human RANTES was produced by human Malavu hepatosarcoma cells, MG-63 osteosarcoma cells or peripheral blood leukocytes (Blood transfusion centers of Antwerp and Leuven) and purified as previously described (Proost, P. et al., (1996) *Methods: A Companion to Methods in Enzymol*. **10**, 82-92; and Proost, P. et al., (1993) *J. Immunol*. **150**, 1000-1010). MCP-2, MCP-3 and GCP-2 were synthesized by Fmoc chemistry (Proost, P. et al., (1995) *Cytokine* **7**, 97-104; and Wuyts, A. et al,. (1997) *Biochemistry* **36**, 2716-2723), recombinant human RANTES was obtained from Peprotech (Rocky Hill, NJ) and recombinant MCP-1 was a gift from Dr. J.J. Oppenheim (NCI-NIH, Frederick, MD).

Human osteosarcoma (HOS) cells transfected with CD4 and one of the CC chemokine receptors CCR1, CCR3 or CCR5 (Deng, H., et al., *Nature* 1996. **381**: 661-666) were grown in DMEM with glutamax. Puromycin (1 µg/ml) was added to the medium as a selection agent. All growth media (Gibco BRL/Life Technologies, Paisley, UK) were enriched with 10% FCS.

Human CD26/DPP IV was obtained from prostasomes, prostate derived organelles, which occur freely in seminal plasma. The enzyme was purified to homogeneity as described before using ion exchange followed by affinity chromatography onto adenosine deaminase (De Meester, I. et al., (1996) *J. Immunol. Methods* **189**, 99-10526).

### Incubation of chemokines with CD26/DPP IV and detection of proteolytic processing

A 100 to 1000 molar excess of chemokine was incubated overnight with CD26/DPP IV in 100 mM Tris/HCl pH 7.7. Chemokines were separated from CD26/DPP IV by SDS-PAGE on a Tris/Tricine gel system as previously described (Proost, P. et al., (1996) *Methods: A Companion to Methods in Enzymol*. **10**, 82-92).

Proteins were electroblotted on PVDF (polyvinylidene fluoride) membranes (Problott, Perkin Elmer, Foster City, CA) and stained with coomassie brilliant blue R250. After destaining, membranes were rinsed at least 5 times with ultrapure water (Milli Q; Millipore, Bedford, MA).

To obtain sufficient amounts of pure truncated chemokine for biological assays, about 50 µg of recombinant chemokine was treated with CD26/DPP IV and the cleavage product was acidified with 0.1 % trifluoroacetic acid (TFA). Tween 20 (0.01 %) was added to prevent the chemokines from sticking to the tubes.

Chemokines were separated from CD26/DPP IV in an acetonitrile gradient on a C-8 Aquapore RP-300 column (1 x 50 mm) (Perkin Elmer). Fractions containing proteins were analyzed by SDS-PAGE and silver stained as described.

CD26/DPP IV treated chemokines, purified by RP-HPLC or excised from PVDF blots, were NH₂-terminally sequenced by Edman degradation on a pulsed liquid phase 477A/120A protein sequencer (Perkin Elmer) using *N*-methylpiperidine as a coupling base.

### Detection of chemotactic activity

Chemokines were tested for their chemotactic potency on freshly isolated peripheral blood neutrophilic granulocytes (10⁶ cells/ml) or cultured monocytic THP-1 cells (0.5 x 10⁶ cells/ml) in the Boyden microchamber (Proost, P. et al., (1996) *Methods: A Companion to Methods in Enzymol*. **10**, 82-92; and Proost, P. et al., (1993) *J. Immunol*. **150**, 1000-1010).

After 45 min (granulocytes) or 2 h (THP-1 cells) incubation at 37 °C, the cells were fixed and stained. The cells that migrated through the 5 µm pore size polycarbonate membranes were counted microscopically in ten oil immersion fields.

The chemotactic index (C.I.) of a sample (triplicates in each chamber) was calculated as the number of cells that migrated to the test sample divided by the number of cells that migrated to control medium. In desensitization experiments, cells were incubated with biologically inactive chemokine-variants for 10 min at 37 °C before transfer to the chamber.

The percentage inhibition of the C.I. obtained by desensitization with HBSS-treated control cells was calculated for the evaluation of chemotaxis desensitization.

### Detection of intracellular Ca²⁺ concentrations

Intracellular Ca²⁺ concentrations ([Ca²⁺]ᵢ were measured as previously described (Wuyts, A., et al., (1997) *Biochemistry* **36**, 2716-2723). Briefly, purified cells were incubated with the fluorescent indicator fura-2 (2.5 µM fura-2/AM, Molecular Probes Europe BV, Leiden, The Netherlands) and 0.01 % Pluronic F-127 (Sigma, St. Louis, MO).

After 30 min, cells were washed twice, resuspended in HBSS with 1 mM Ca²⁺ and incubated for 10 min at 37 °C before fura-2 fluorescence was measured in an LS50B luminescence spectrophotometer (Perkin Elmer). Upon excitation at 340 and 380 nm, fluorescence was detected at 510 nm. The [Ca²⁺]ᵢ was calculated from the Grynkiewicz equation (Grynkiewicz, G. et al. (1985) *J. Biol. Chem*. **260**, 3440-3450).

In order to determine *R*ₘₐₓ, the cells were lysed with 50 µM digitonin. Subsequently, the pH was adjusted to 8.5 with 20 mM Tris and *R*ₘᵢₙ was obtained by addition of 10 mM EGTA to the lysed cells. The *K*_{d} used for calibration was 224 nM. For desensitization experiments, cells were first stimulated with buffer or chemokine at different concentrations. As a second stimulus, chemokines were added at a concentration inducing a significant increase in the [Ca²⁺]ᵢ after prestimulation with buffer. The percentage inhibition of the [Ca²⁺]ᵢ-increase in response to the second stimulus by prestimulation of the cells was calculated.

### Inhibition of HIV-1 infection

The HIV-1 M-tropic strain BaL was obtained through the MAC AIDS reagent project (Herts, UK). Peripheral blood mononuclear cells (PBMC) from healthy donors were isolated by density gradient centrifugation (5,23) and stimulated with PHA at 1 µg/ml (Sigma, Bornem, Belgium) for 3 days at 37°C. The activated cells (PHA-stimulated blasts) were washed three times with PBS, and infected with a virus as described previously (Schols D. et al., *J. Exp. Med*., **186**, 1383-1388, 1997). HIV-1 infected or mock-infected PHA-stimulated blasts were cultured in the presence of 25 U/ml of IL-2 and varying concentrations of RANTES (1-68) or RANTES (3-68). Cell supernatant was collected at day 10 and HIV-1 core antigen in the supernatant was analysed by a p-24 Ag ELISA kit (DuPont/NEN Life Science Products, Brussels, Belgium).

### Results

### Identification and biological characterization of natural NH₂-terminally truncated RANTES.

A different NH₂-terminally truncated form of human GCP-2 has been previously isolated (Proost, P. et al. (1993) *J. Immunol*. **150**, 1000-1010). The least truncated GCP-2-form was cleaved beyond Pro at the penultimate position [GCP-2(3-75)]. Using a similar standard purification procedure (cfr. also MCP-2, *vide supra*), the C-C chemokine RANTES was purified from peripheral blood leukocytes or sarcoma cells (Proost, P. et al., (1996) *Methods: A Companion to Methods in Enzymol*. **10**, 82-92).

In particular, conditioned media from MG-63 or Malavu sarcoma cells induced with a cytokine mixture were fractionated to isolate natural chemokine variants. The chemokines were purified by subsequent antibody or heparin affinity chromatography, cation-exchange chromatography (mono S FPLC) and RP-HPLC, and immunoreactive forms were detected by specific chemokine ELISAs. On the cation-exchange column, IL-8 was found to elute in close proximity of RANTES (between 0.7 and 0.75 M NaCl). Nevertheless, both chemokines were separated from each other by RP-HPLC (RANTES and IL-8 eluting at 27.5% and 30% acetonitrile, respectively). Amino acid sequence analysis of the pure proteins confirmed that IL-8 occured in different NH₂-terminally truncated forms, which were previously isolated on the basis of their chemotactic activity (Van Damme J. et al., Fur. J. Biochem., 1989, 181, 337-344). However, for RANTES only one single form was isolated, which was missing two NH₂-terminal residues compared to intact RANTES. In view of its predominant appearance, this RANTES(3-68) was analyzed in more detail to verify its chemotactic activity for monocytes and eosinophils. In particular, RANTES(3-68) was tested for chemotactic and/or intracellular Ca²⁺-releasing activity and their biological potency was compared with that of the respective intact chemokines.

NH₂-terminal deletion of two residues from RANTES resulted in considerably decreased monocyte chemotactic and Ca²⁺-releasing activities. Compared to intact natural RANTES (minimal effective dose of 3-10 ng/ml), natural RANTES(3-68) was totally inactive when tested at concentrations as high as 300 ng/ml in the Boyden microchamber (Fig. 5). In addition, 10 times higher concentrations of natural RANTES(3-68), compared to RANTES(1-68), were necessary to obtain a similar Ca²⁺-response (Fig. 6).

### CD26/DPP IV removes the NH₂-terminal dipeptides of chemokines

In order to investigate whether the aminopeptidase CD26/DPP IV could be responsible for the NH₂-terminal truncation of RANTES, the intact chemokine was incubated overnight with CD26/DPP IV, blotted to PVDF membranes, stained with Coomassie blue and subjected to automatic Edman degradation. CD26/DPP IV treatment of RANTES resulted in the removal of the NH₂-terminal dipeptides. Parallel incubation of chemokine with buffer without CD26/DPP IV had no effect.

Since other chemokines contained the consensus sequence for CD26/DPP IV cleavage and since the NH₂-terminus of MCPs was shown to be crucial for biological activity (Gong, J. et al. (1996) *J. Biol. Chem*. **271**, 10521-10527; and Gong, J. et al. (1995) *J. Exp. Med*. **181**, 631-640), MCP-1, MCP-2 and MCP-3 were also incubated with CD26/DPP IV.

Alter treatment, MCPs were blotted on PVDF membranes and Coomassie blue stained to confirm that a sufficient amount of protein was recovered for Edman degradation.

However, no NH₂-terminal sequence could be detected, indicating that CD26/DPP IV does not alter the NH₂-terminus of MCPs which is blocked for Edman degradation by a pyroglutamic acid.

### Comparison of the biological activity of intact and CD26/DPP IV-treated RANTES.

Similar to natural RANTES(3-68), C-8 RP-HPLC purified, CD26/DPP IV-treated recombinant RANTES was inactive in Boyden microchamber chemotaxis experiments when used at concentrations up to 1 µg/ml, while a significant monocyte chemotactic response was detected with intact recombinant RANTES from 30 to 100 ng/ml onwards (Fig. 5).

When the truncation effect was tested in the Ca²⁺-mobilization assay, RANTES(3-68) induced a low but significant increase at 100 ng/ml. Intact RANTES, however, was already active at 10 ng/ml (Fig. 6). In conclusion, although only two NH₂-terminal residues were removed, the monocyte chemotactic and Ca²⁺-mobilizing potency of RANTES decreased 10 to 100-fold.

### RANTES(3-68) is a natural chemotaxis antagonist for intact RANTES

In view of the inactivity of RANTES(3-68) in monocyte chemotaxis experiments, we tested whether this truncated RANTES might act as an antagonist. RANTES(3-68), at 1 µg/ml, almost completely (82 %) desensitized for the chemotactic effect of 100 ng/ml of intact RANTES (Table III).

When a 3-fold excess of RANTES(3-68) was added to the upper well, chemotaxis of THP-1 cells towards intact RANTES was inhibited by about 50-70 %. RANTES(3-68) at 300 ng/ml could still inhibit about 30 % of the chemotactic response towards an equal concentration of intact RANTES.

In Ca²⁺-mobilization experiments with THP-1 cells (Fig. 7), 30 ng/ml of intact RANTES could desensitize for the effect of 30 ng/ml of intact RANTES for 39 ±5 %. About ten-fold higher concentrations of RANTES(3-68) were necessary to obtain the same amount of desensitization. However, at 300 ng/ml, RANTES(3-68) by itself gave a significant Ca²⁺-response. This Ca²⁺-response was comparable to the response obtained with 30 ng/ml of intact RANTES.

**TABLE III**

| *RANTES(3-68) desensitizes monocyte chemotaxis induced by RANTES(1-68)*^{*1*} | | | | | | | |
|---|---|---|---|---|---|---|---|
| Chemokine(ng/ml) | | Chemotactic response (CI) | | | | | |
| Lower well | Upper well | | | | | | % inhibition |
| RANTES (1-68) | RANTES (3-68) | A | B | C | D | mean ± SEM | mean ± SEM |
| 300 | 1000 | 12.5 | 7.5 | 27.5 | 50.5 | 25 ± 10 | 67 ± 8 |
| | 300 | 22.0 | 20.5 | 72.5 | 79.5 | 49 ± 16 | 31 ±13 |
| | 0 | 41.0 | 46.0 | 71.5 | 97.0 | 64 ±1 3 | 0 |
| 100 | 1000 | 4.0 | 3.0 | 13.5 | 11.0 | 8 ± 3 | 82 ± 4 |
| | 300 | 7.5 | 7.0 | 29.0 | 33.0 | 19 ± 7 | 53 ± 11 |
| | 0 | 24.0 | 21.5 | 50.0 | 44.5 | 35 ± 7 | 0 |

| | | | | | | | |
|---|---|---|---|---|---|---|---|
| ¹ Results represent the chemotactic index (C.I.) of four (A to D) independent experiments (including mean ± SEM) and the percentage (%) inhibition (mean ± SEM of the % inhibition of the four experiments) of the chemotactic response towards RANTES(1-68) after preincubation of the THP-1 cells with inactive RANTES(3-68) or buffer. | | | | | | | |

### Impaired chemotactic activity of RANTES(3-68) for human monocytes and eosinophils

In Table IV the chemotactic potency of natural RANTES(3-68) is compared with that of the monocyte chemotactic protein MCP-3 and intact RANTES(1-68). It can be seen that MCP-3 and RANTES(1-68) are still chemotactic for freshly isolated peripheral blood monocytes at 3 ng/ml and 30 ng/ml, respectively, whereas natural RANTES(3-68) remained inactive at 100 ng/ml.

The reduced chemotactic potency of this natural variant, was confirmed with recombinant RANTES(3-68). Although weakly chemotactic for monocytes (at 1 µg/ml), purified recombinant RANTES(3-68) showed a specific activity which is 10-fold lower than that of intact recombinant RANTES.

Finally, the chemotactic potency of RANTES(3-68) was verified on human eosinophils, which were still responsive to 100 ng/ml of intact RANTES and 30 ng/ml of MCP-3 (Fig. 8). Similar to monocytes, eosinophil migration was only stimulated by RANTES(3-68) at 1 µg/ml.

**TABLE IV**

| *Comparison of the monocyte chemotactic activity of RANTES(3-68) with RANTES(1-68) and MCP-3* | | | | |
|---|---|---|---|---|
| | monocyte chemotactic activity ^{a)} | | | |
| conc. (ng/ml) | MCP-3 | natRANTES (3-68) | recRANTES (1-68) | recRANTES (3-68) |
| 1000 | -^{b)} | - | 3.6 ± 0.8 (6) | 3.3 (1) |
| 300 | 6.0 ± 1.2 (6) | - | - | - |
| 100 | - | 1.1 ± 0.1 (3) | 3.3 ± 0.4 (6) | 1.0 (1) |
| 30 | 6.9 ± 1.0 (6) | 1.7 ± 0.2 (3) | - | - |
| 10 | - | 1.9 ± 0.6 (3) | 1.9 ± 0.4 (6) | < 1.0 (1) |
| 3 | 4.1 ± 0.4 (6) | - | - | - |

| | | | | |
|---|---|---|---|---|
| ^{a)} mean chemotactic index (CI) ± SEM (n) on freshly isolated peripheral blood monocytes. | | | | |
| ^{b)} not determined | | | | |

### RANTES(3-68) signals and desensitizes for RANTES(1-68) through CCR5, but not through CCR1 and CCR3

To explain the reduced chemotactic activity of RANTES(3-68), the capacity of this chemokine variant to bind and signal through the known receptors used by RANTES was verified.

HOS cells transfected with the chemokine receptors CCR1, CCR3 or CCR5 were used in a signaling assay measuring increases in the intracellular calcium concentration. At concentrations up to 300 ng/ml, RANTES(3-68) did not increase the [Ca²⁺]ᵢ in HOS cells transfected with CCR1 (Table V) or CCR3 (data not shown), whereas 30 ng/ml and 100 ng/ml of intact RANTES was sufficient to induce an increase in [Ca²⁺]ᵢ in CCR1 and CCR3 transfectants, respectively.

However, both intact and truncated RANTES were able to induce a significant rise in [Ca²⁺]ᵢ in CCR5 transfectants at 30 ng/ml. Furthermore, by pre-incubation of CCR5-transfected cells with a 3 to 10-fold excess of either RANTES(3-68) or intact RANTES, an equal inhibition (about 75%) of the [Ca²⁺]ᵢ rise by a subsequent challenge with intact RANTES (100 ng/ml) was obtained (Fig. 9).

In contrast, 300 ng/ml of RANTES(3-68) only marginally desensitized the calcium response of CCR1 and CCR3-transfected cells to 100 ng/ml of intact RANTES, whereas a 3-fold excess of intact RANTES as first stimulus almost completely inhibited the [Ca²⁺]ᵢ rise in these cells by subsequent RANTES(1-68). It must be concluded that removal of two NH₂-terminal residues from RANTES has a significant impact on signal transduction in that the chemokine receptors CCR1 and CCR3 are no longer functionally recognized. Therefore, the impaired chemotactic potency of RANTES(3-68) can be explained by its inability to function through CCR1 and CCR3. In contrast, RANTES(3-68) fully retained the CCR5 signalling characteristic of intact RANTES. RANTES(3-68) can be anti-inflammatory by competing with intact RANTES, but may still function as an HIV-inhibitor by retaining its capacity to bind CCR5.

**TABLE V**

| *Calcium mobilization by RANTES forms in CCR1 and CCR5 transfectants* | | | |
|---|---|---|---|
| chemokine | conc. (ng/ml) | increase in [Ca²⁺]ᵢ (nM)^{a)} | |
| | | CCR1 | CCR5 |
| RANTES(1-68) | 300 | 133 ± 5 (3) | 96 ± 1 (2) |
| | 100 | 100 ± 28 (3) | 60 ± 4 (2) |
| | 30 | 25 ± 8 (3) | 24 ± 2 (2) |
| | 10 | <15 (2) | <15 (1) |
| RANTES(3-68) | 300 | 19 ± 9 (3) | 119 ± 5 (2) |
| | 100 | <15 ± 0 (3) | 76 ± 4 (2) |
| | 30 | <15 (2) | 56 ± 13 (2) |
| | 10 | | <15 (1) |

| | | | |
|---|---|---|---|
| ^{a)} the mean increase in [Ca²⁺]ᵢ in nM ± SEM of two or more independent experiments is shown. | | | |

### Inhibition of CC chemokine-induced chemotaxis by RANTES(3-68) in human monocytic cells

To verify whether inhibition of CC chemokine signaling by RANTES(3-68) also occurred in monocytic cells, inhibition experiments were conducted in THP-1 cells. It was evidenced that RANTES(3-68) showed a 10-fold reduction in potency to increase the [Ca²⁺]ᵢ in monocytic cells compared to intact RANTES (data not shown). In addition, the chemotactic effect of intact RANTES (30 ng/ml) on monocytic cells was inhibited (71%) by incubating the test cells with 300 ng/ml RANTES(3-68) as shown in Table VI.

Furthermore, RANTES(3-68) reduced the chemotactic response to other CC chemokines, including monocyte chemotactic protein-3 (MCP-3) (67%), macrophage inflammatory protein-1a (MIP-1α) (61%) and MIP-1β (80%).

This illustrates that RANTES(3-68) functions as a broad spectrum inhibitor of monocytic cell migration induced by other CC chemokines.

**TABLE VI**

| *Inhibition of monocytic cell chemotaxis towards CC chemokines by RANTES(3-68)* | | | | |
|---|---|---|---|---|
| | | inhibition of THP-1 cell chemotaxis | | |
| chemokine^{a)} | conc. (ng/ml) | buffer^{b}^{,}^{c)} | RANTES(3-68)^{b}^{,}^{c)} | % inhibition^{d)} |
| RANTES | 30 | 19.0 ± 6.6 | 3.7 ± 0.6 | 71 ± 16 |
| MCP-3 | 30 | 48.5 ± 9.3 | 24.9 ± 2.0 | 45 ± 10 |
| MCP-3 | 3 | 7.6 ± 2.5 | 3.1 ± 0.8 | 67 ± 13 |
| MIP-1α | 30 | 6.2 ± 2.4 | 3.0 ± 1.1 | 61 ± 22 |
| MIP-1β | 300 | 4.3 ± 1.0 | 1.9 ± 0.6 | 80 ± 12 |
| control | | 1.5 ± 0.5 | 1.0 ± 0.5 | |

| | | | | |
|---|---|---|---|---|
| a) RANTES, MCP-3, MIP-1a, MIP-1b and buffer were added as chemoattractants to the lower wells of the microchamber. | | | | |
| b) the upper wells of the microchamber were filled with THP-1 cells preincubated (10 min, 37°C) with 300 ng/ml RANTES(3-68) or with buffer. | | | | |
| c) mean CI ± SEM of four independent experiments. | | | | |
| d) inhibition of migration induced by intact chemokines in the presence of RANTES(3-68) at 300 ng/ml. | | | | |

### RANTES (3-68) is a more potent HIV-1 inhibitor than RANTES (1-68)

RANTES (3-68) and RANTES (1-68) were compared for their ability to inhibit HIV-1 infection of peripheral blood mononuclear cells with M-tropic Ba-L strain (see Figure 10). RANTES was added to the cultures at the time of infection and p-24 Ag concentrations were determined in the culture supernatant 10 days after the infection. Inhibition of HIV-1 infection by 40 ng/ml of RANTES (3-68) was significantly better (91% inhibition) than the inhibition obtained with an equal concentration of intact RANTES (60% inhibition) (p<0.01 with a Student's t test). In two out of four experiments, p-24 was still detected after treatment with 1 µg/ml of intact RANTES, whereas all PBMC cultures remained uninfected when pre-treated wit 1 µg/ml of RANTES (3-68). In conclusion, although RANTES (3-68) was less efficient compared to intact RANTES in Ca²⁺ -mobilization or chemotaxis assays, NH₂-terminal truncation of RANTES by CD26/DPP IV significantly enhanced its anti-HIV-1 activity. At the same time, RANTES (3-68), which is a much weaker chemotaxis agonist compared to intact RANTES, can act as a negative feedback to weaken the inflammatory response.

## Claims

1. Amino-terminally truncated C-C chemokines, having chemokine antagonistic activity.

2. Amino-terminally truncated C-C chemokines, according to claim 1, missing up to 5 amino-terminally amino acids and having a chemokine antagonistic activity

3. Amino-terminally truncated C-C chemokines, according to any of the preceding claims selected among the group consisting of MCP-1, MCP-2, MCP-3, MCP-4, MCP-5 and RANTES.

4. Amino-terminally truncated MCP-2(6-76), which is MCP-2 missing the 5 NH₂-terminal amino acids, as shown in Figure 1.

5. Amino-terminally truncated RANTES(3-68), which is RANTES missing the first 2 amino acids, as shown in Figure 1.

6. Amino-terminally truncated C-C chemokines according to one or more of the preceding claims, in a glycosylated form.

7. DNA molecules comprising the DNA sequences coding for the amino-terminally truncated chemokines of the invention according to one or more of the preceding claims, including nucleotide sequences substantially the same

8. An expression vector which comprises the DNA molecule of any claim 7.

9. A host cell comprising the expression vector of claim 8.

10. A recombinant process for preparing any of the proteins from claim 1 to 5, comprising culturing in an appropriate culture medium the cells of claim 9.

11. A protein according to any of the claims from 1 to 6 for use as medicament.

12. Use of a protein according to any of the claims from 1 to 6, in the manufacture of a medicament for the therapy and/or diagnosis of diseases, in which an antagonistic activity of the chemokine effects is required.

13. Use according to claim 12, in the manufacture of a medicament for the treatment of inflammatory diseases, HIV-infection, angiogenisis- and hematopoiesis-related diseases, and tumors.

14. A pharmaceutical composition comprising the protein according to any of the claims from 1 to 6 together with one or more pharmaceutically acceptable carriers and/or excipients.

15. Use of CD26/DPP IV in the therapy and/or diagnosis of the diseases, in which an antagonistic activity of the chemokine effects is required.

16. Use according to claim 15, for the treatment of inflammatory, immune and infectious diseases.


SEQUENCE LISTING

(1) GENERAL INFORMATION:

(i) APPLICANT:
(A) NAME: APPLIED RESEARCH SYSTEMS ARS HOLDING N.V.
(B) STREET: 14 JOHN B. GORSIRAWEG
(C) CITY: CURACAO
(E) COUNTRY: THE NETHERLANDS ANTILLES
(F) POSTAL CODE (ZIP): NONE
(G) TELEPHONE: 599-9-639300
(H) TELEFAX: 599-9-614129

(ii) TITLE OF INVENTION: AMINO-TERMINAL TRUNCATED C-C CHEMOKINES AS
CHEMOKINE ANTAGONISTS

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO: 1:

(i) SEQUENCE CHARACTERISTICS:
(A) LENGTH: 91 amino acids
(B) TYPE: amino acid
(C) STRANDEDNESS:
(D) TOPOLOGY: linear

(ii) MOLECULE TYPE: protein

(iii) HYPOTHETICAL: NO

(iv) ANTI-SENSE: NO

(ix) FEATURE:
(A) NAME/KEY: Protein
(B) LOCATION:1..68

(xi) SEQUENCE DESCRIPTION: SEQ ID NO: 1:

Met Lys Val Ser Ala Ala Arg Leu Ala Val Ile Leu Ile Ala Thr Ala
-20 -15 -10

Leu Cys Ala Pro Ala Ser Ala Ser Pro Tyr Ser Ser Asp Thr Thr Pro
-5 1 5

Cys Cys Phe Ala Tyr Ile Ala Arg Pro Leu Pro Arg Ala His Ile Lys
10 15 20 25

Glu Tyr Phe Tyr Thr Ser Gly Lys Cys Ser Asn Pro Ala Val Val Phe
30 35 40

Val Thr Arg Lys Asn Arg Gln Val Cys Ala Asn Pro Glu Lys Lys Trp
            45                  50                  55

Val Arg Glu Tyr Ile Asn Ser Leu Glu Met Ser
        60                  65

(2) INFORMATION FOR SEQ ID NO: 2:

(i) SEQUENCE CHARACTERISTICS:
(A) LENGTH: 66 amino acids
(B) TYPE: amino acid
(C) STRANDEDNESS:
(D) TOPOLOGY: linear

(ii) MOLECULE TYPE: protein

(iii) HYPOTHETICAL: NO

(iv) ANTI-SENSE: NO

(xi) SEQUENCE DESCRIPTION: SEQ ID NO: 2:

Tyr Ser Ser Asp Thr Thr Pro Cys Cys Phe Ala Tyr Ile Ala Arg Pro
1               5                   10                  15

Leu Pro Arg Ala His Ile Lys Glu Tyr Phe Tyr Thr Ser Gly Lys Cys
            20                  25                  30

Ser Asn Pro Ala Val Val Phe Val Thr Arg Lys Asn Arg Gln Val Cys
        35                  40                  45

Ala Asn Pro Glu Lys Lys Trp Val Arg Glu Tyr Ile Asn Ser Leu Glu
    50                  55                  60

Met Ser
65

(2) INFORMATION FOR SEQ ID NO: 3:

(i) SEQUENCE CHARACTERISTICS:
(A) LENGTH: 99 amino acids
(B) TYPE: amino acid
(C) STRANDEDNESS:
(D) TOPOLOGY: linear

(ii) MOLECULE TYPE: protein

(iii) HYPOTHETICAL: NO

(ix) FEATURE:
(A) NAME/KEY: Protein
(B) LOCATION:1..76

(xi) SEQUENCE DESCRIPTION: SEQ ID NO: 3:

Met Lys Val Ser Ala Ala Leu Leu Cys Leu Leu Leu Met Ala Ala Thr

-20                 -15                 -10

Phe Ser Pro Gln Gly Leu Ala Gln Pro Asp Ser Val Ser Ile Pro Ile
        -5                  1                   5

Thr Cys Cys Phe Asn Val Ile Asn Arg Lys Ile Pro Ile Gln Arg Leu
10                  15                  20                  25

Glu Ser Tyr Thr Arg Ile Thr Asn Ile Gln Cys Pro Lys Glu Ala Val
                30                  35                  40

Ile Phe Lys Thr Lys Arg Gly Lys Glu Val Cys Ala Asp Pro Lys Glu
            45                  50                  55

Arg Trp Val Arg Asp Ser Met Lys His Leu Asp Gln Ile Phe Gln Asn
        60                  65                  70

Leu Lys Pro
    75

(2) INFORMATION FOR SEQ ID NO: 4:

(i) SEQUENCE CHARACTERISTICS:
(A) LENGTH: 71 amino acids
(B) TYPE: amino acid
(C) STRANDEDNESS:
(D) TOPOLOGY: linear

(ii) MOLECULE TYPE: protein

(iii) HYPOTHETICAL: NO

(xi) SEQUENCE DESCRIPTION: SEQ ID NO: 4:

Ser Ile Pro Ile Thr Cys Cys Phe Asn Val Ile Asn Arg Lys Ile Pro
1               5                   10                  15

Ile Gln Arg Leu Glu Ser Tyr Thr Arg Ile Thr Asn Ile Gln Cys Pro
            20                  25                  30

Lys Glu Ala Val Ile Phe Lys Thr Lys Arg Gly Lys Glu Val Cys Ala
        35                  40                  45

Asp Pro Lys Glu Arg Trp Val Arg Asp Ser Met Lys His Leu Asp Gln
    50                  55                  60

Ile Phe Gln Asn Leu Lys Pro
65                  70